# EUROPEAN PATENT APPLICATION

(11) **EP 3 944 776 A1**
(43) Date of publication of application: **02.02.2022**
(21) Application number: 20188203.2
(22) Date of filing: 28.07.2020
(51) Int. Cl.: A24F 40/42, A24F 40/465, H05B 6/44

(54) **HEATING SYSTEM FOR AN AEROSOL GENERATION ASSEMBLY AND ASSOCIATED CARTRIDGE, AEROSOL GENERATION DEVICE AND AEROSOL GENERATION ASSEMBLY**

(71) Applicant: JT International S.A., 1202 Genève (CH)
(72) Inventor: POPOOLA, Ola, Walton-on-Thames, KT12 2SJ (GB); WRIGHT, Alec, Chertsey, KT16 9BE (GB); ROGAN, Andrew, Forres, Moray, IV36 2ZH (GB); ADAIR, Kyle, Lisburn, BT28 2UW (GB); LOVEDAY, Peter, Woking, Surrey, GU212EL (GB)
(74) Representative: Lavoix

(57) **Abstract**

The present invention concerns a heating system (34) for an aerosol generation assembly comprising an aerosol generation device and a cartridge. The heating system (34) comprising a magnetic core (84) disposed at least partially around a storage portion (66) of the cartridge, a primary and a secondary windings (81, 82) wound around the magnetic core (84) on opposite sides of the magnetic core (84), a load (83) connected electrically to the secondary winding (82), an AC source (77) connected electrically to the primary winding (81) and able to generate an alternative current, and a heating control module (75) connecting a battery and the AC source (77), and configured to control the operation of the AC source (77) to generate aerosol by heat transfer from the magnetic core (84) to the aerosol forming precursor contained in the storage portion (66).

## Description

### FIELD OF THE INVENTION

The present invention concerns a heating system for an aerosol generation assembly.

The present invention also concerns an associated cartridge, an aerosol generation device designed to operate with such a cartridge and an aerosol generation assembly comprising such a cartridge and such an aerosol generation device.

### BACKGROUND OF THE INVENTION

Different types of aerosol generation devices are already known in the art. Generally, such devices comprise a storage portion for storing an aerosol forming precursor, which can comprise for example a liquid or a solid. A heating system is formed of one or more electrically activated resistive heating elements arranged to heat said precursor to generate the aerosol. The aerosol is released into a flow path extending between an inlet and outlet of the device. The outlet may be arranged as a mouthpiece, through which a user inhales for delivery of the aerosol.

In some aerosol generation devices, the precursor is stored in a removable cartridge. Thus, when the precursor is consumed, the cartridge can be easily removed and replaced. In order to attach the removable cartridge to the device body, a screw-threaded connection can for example be used.

In some known aerosol generation devices using removable cartridges, the heating system is distributed between the aerosol generation device and the cartridge. In this case, the part of the heating system comprised in the aerosol generation device may be formed for example by a pair of contacts connected electrically to the battery. The part of the heating system comprised in the cartridge can be for example formed by a resistance wound around a wick and a pair of contacts cooperating with the corresponding pair of the contacts of the aerosol generation device to connect electrically the resistance to the battery. In variant, the cartridge can be connected wirelessly to the aerosol generation device. In this case, the contacts are replaced by a primary and a secondary winding integrated respectfully into the aerosol generation device and the cartridge to generate an electric current for the resistance, by magnetic induction. According to some other examples, heat can be transmitted directly between the cartridge and the aerosol generation device using for example heating plates integrated in both device and cartridge.

One can conceive that the heating system distribution, as proposed in the art, between an aerosol generation device and a removable cartridge increases the structure complexity and the cost of both elements. This requires adding additional components notably into the cartridge that increases its structure and cost and make more difficult its recycling.

### SUMMARY OF THE INVENTION

One of the aims of the present invention is to propose a heating system adapted to be used by an aerosol generation device and a removable cartridge without increasing structure complexity and costs of these elements. The invention also allows cartridge recycling in a simple way.

For this purpose, the invention relates to a heating system for an aerosol generation assembly comprising an aerosol generation device and a cartridge designed to operate with the aerosol generation device, the aerosol generation device comprising a battery and the cartridge comprising a storage portion for storing an aerosol forming precursor and a cartridge housing delimiting the storage portion.

The heating system comprises:
- a magnetic core disposed at least partially around the storage portion ;
- a primary and a secondary winding wound around the magnetic core on opposite sides of the magnetic core;
- a load connected electrically to the secondary winding;
- an AC source connected electrically to the primary winding and able to generate an alternative current;
- a heating control module connecting the battery and the AC source, and configured to control the operation of the AC source to generate aerosol by heat transfer from the magnetic core to the aerosol forming precursor contained in the storage portion.

Indeed, using these features, it is possible to heat the precursor stored in the storage portion by currents passing through the magnetic core. Thus, there is no need to provide special internal components (as a wick or resistance) inside the cartridge. Hence, the cartridge structure can be simplified which decreases the cost of the cartridge and makes its recycling simpler.

According to some embodiments, the heating control module is configured to control the operation of the AC source based on the precursor temperature and/or the time of operating and/or the rate of the aerosol flow.

Thanks to these features, it is possible to control the operation of the aerosol generation device by controlling the operation of the AC source.

According to some embodiments, the magnetic core forms a closed shape.

Thanks to these features, the precursor can be heated by a major part of the external surface of the magnetic core.

According to some embodiments, the magnetic core forms a rectangular shape comprising a first pair of parallel sides and a second pair of parallel sides.

Thanks to these features, the magnetic core may be arranged around a storage portion having rectangular or cylindrical shape.

According to some embodiments, the magnetic core presents a fixed magnetic core part integrated into the aerosol generation device and a removable magnetic core part integrated into the cartridge, the removable magnetic core part being in connection with the fixed magnetic core part when the cartridge is inserted into the aerosol generation device.

Thanks to these features, at least a part of the magnetic core can be integrated into the cartridge.

According to some embodiments, the removable magnetic core part forms at least partially the cartridge housing and presents a contact surface in contact with the aerosol forming precursor.

Thanks to these features, the precursor can be heated directly by contact with the corresponding part of the magnetic core.

According to some embodiments, the primary and the secondary windings are wound around the fixed magnetic core part.

Thanks to these features, it is possible to arrange the windings inside the aerosol generation device.

According to some embodiments, the fixed magnetic core part and the removable magnetic core part form respectively the first pair of parallel sides and the second pair of parallel sides.

Thanks to these features, it is possible to integrate two parallel sides of the magnetic core into the cartridge.

According to some embodiments, the primary and the secondary windings, the load, the AC source and the heating control module are integrated into the aerosol generation device.

Thanks to these features, it is possible to integrate into the cartridge only a small number of elements so as to simplify its structure.

The invention also relates to a cartridge designed to operate with an aerosol generation device and comprising:
- a storage portion for storing an aerosol forming precursor;
- a cartridge housing delimiting the storage portion;
- a removable magnetic core part of the magnetic core of the heating system as described above.

According to some embodiments, the cartridge housing is extending along a cartridge axis and said removable magnetic core part is integrated into the cartridge housing and extends along the cartridge axis.

Thanks to these features, the precursor stored into the cartridge can be heated by direct heat transfer from the magnetic core.

The invention also relates to an aerosol generation device designed to operate with a cartridge and comprising:
- a battery;
- a fixed magnetic core part of the magnetic core of the heating as described above.

According to some embodiments, the aerosol generation device is extending along a device axis coinciding with a cartridge axis when a cartridge is inserted into the aerosol generation device and said fixed magnetic core part extends perpendicularly to the device axis.

Thanks to these features, a cartridge of rectangular or cylindrical shape can be used with the aerosol generation device.

The invention also relates to an aerosol generation assembly comprising a cartridge as described above and an aerosol generation device as described above.

### BRIEF DESCRIPTION OF THE DRAWINGS

The invention and its advantages will be better understood upon reading the following description, which is given solely by way of non-limiting example and which is made with reference to the appended drawings, in which:
- Figure 1 is a schematic diagram showing an aerosol generation assembly according to the invention, the assembly comprising a heating system according to the invention;
- Figure 2 is a schematic diagram showing in more detail the heating system of Figure 1; and
- Figure 3 is a schematic diagram showing the operation of the heating system of Figure 1.

### DETAILED DESCRIPTION OF THE INVENTION

Before describing the invention, it is to be understood that it is not limited to the details of construction set forth in the following description. It will be apparent to those skilled in the art having the benefit of the present disclosure that the invention is capable of other embodiments and of being practiced or being carried out in various ways.

As used herein, the term "**aerosol generation device**" or "**device**" may include a vaping device to deliver an aerosol to a user, including an aerosol for vaping, by means of aerosol generating unit (e.g. an aerosol generating element which generates vapor which condenses into an aerosol before delivery to an outlet of the device at, for example, a mouthpiece, for inhalation by a user). The device may be portable. "Portable" may refer to the device being for use when held by a user. The device may be adapted to generate a variable amount of aerosol, e.g. by activating a heater system for a variable amount of time (as opposed to a metered dose of aerosol), which can be controlled by a trigger. The trigger may be user activated, such as a vaping button and/or inhalation sensor. The inhalation sensor may be sensitive to the strength of inhalation as well as the duration of inhalation to enable a variable amount of vapor to be provided (so as to mimic the effect of smoking a conventional combustible smoking article such as a cigarette, cigar or pipe, etc.). The device may include a temperature regulation control to drive the temperature of the heater and/or the heated aerosol generating substance (aerosol pre-cursor) to a specified target temperature and thereafter to maintain the temperature at the target temperature that enables efficient generation of aerosol.

As used herein, the term "**aerosol**" may include a suspension of precursor as one or more of: solid particles; liquid droplets; gas. Said suspension may be in a gas including air. Aerosol herein may generally refer to/include a vapor. Aerosol may include one or more components of the precursor.

As used herein, the term "**aerosol-forming precursor**" or "**precursor**" or "**aerosol-forming substance**" or "**substance**" may refer to one or more of a: liquid; solid; gel; mousse; foam or other substances. The precursor may be processable by the heating system of the device to form an aerosol as defined herein. The precursor may comprise one or more of: nicotine; caffeine or other active components. The active component may be carried with a carrier, which may be a liquid. The carrier may include propylene glycol or glycerin. A flavoring may also be present. The flavoring may include Ethylvanillin (vanilla), menthol, Isoamyl acetate (banana oil) or similar. A solid aerosol forming substance may be in the form of a rod, which contains processed tobacco material, a crimped sheet or oriented strips of reconstituted tobacco (RTB).

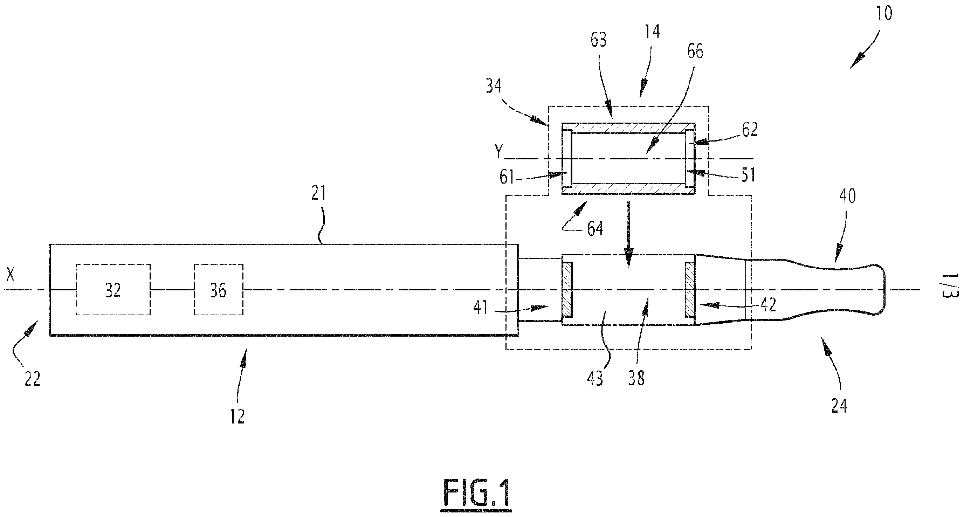

Referring to Figure 1, an aerosol generation assembly 10 according to the invention comprises an aerosol generation device 12 and a removable cartridge 14.

The aerosol generation device 12 comprises a device housing 21 extending between a battery end 22 and a mouthpiece end 24 along a device axis X.

The device housing 21 delimits an interior part of the aerosol generation device 12 comprising a power block 32 designed to power the device 12, at least a part of a heating system 34 powered by the power block 32, and a controller 36. The device housing 21 also defines a payload compartment 38 which may be arranged in the interior part of the device 12 or/and defined at least partially by at least one wall of the device housing 21. Additionally, on the mouthpiece end 24, the device housing 21 defines a mouthpiece 40. The mouthpiece 40 is in a fluid communication with the payload compartment 38 and defines an airflow outlet configured to deliver aerosol to the user when the aerosol generation device 12 is operated with the cartridge 14. The device housing 21 may further comprise other internal components performing different functionalities of the device 12 known in the art.

It should be noted that Figure 1 presents only a schematic diagram of different components of the aerosol generation device 12 and does not necessarily show the real physical arrangement and dimensions of these components. Particularly, such an arrangement can be chosen according to the design of the aerosol generation device 12 and technical features of its components.

The power block 32 comprises a battery and a battery charger. The battery is for example a known battery designed to be charged using the power supply furnished by an external source and to provide a direct current of a predetermined voltage. The battery charger is able to connect the battery to the external source and comprises for this purpose a power connector (like for example a mini-USB connector) or wireless charging connector. The battery charger is also able to control the power delivered from the external source to the battery according for example a predetermined charging profile. Such a charging profile can for example define a charging voltage of the battery depending on its level of charge.

The controller 36 is formed for example by a microcontroller and is able to control the operation of the aerosol generation device 12 by controlling the operation of its different components.

The payload compartment 38 defines a cavity designed to receive the cartridge 14. In the example of figure 1, the payload compartment 38 extends along the device axis X between a pair of parallel walls 41, 42 of the device housing 21. In the same example, the payload compartment 38 is further delimited by at least one back wall 43 extending between the parallel walls 41, 42 along the device axis X. In this case, the payload compartment 38 may further define at least one side opening opposite to the back wall 43 and used to insert the cartridge 44 into the payload compartment 38. The opening may be for example adapted to be covered by an appropriate cover.

Each of the parallel walls 41, 42 is for example perpendicular to the device axis X. The wall 41 is adjacent to the battery end 22 and defines a hole suitable for an airflow passage between an airflow channel formed inside the device housing 21 and the cartridge 14. The wall 42 is adjacent to the mouthpiece end 24 and defines a hole suitable for an airflow passage between the cartridge 14 and the airflow outlet of the mouthpiece 40.

The cartridge 14 comprises a cartridge housing 51 and the part of the heating system 34 not comprised in the aerosol generation device 12 as it will be explained below in further detail. The cartridge housing 51 defines two parallel walls 61, 62 and extends between these walls 61, 62 along a cartridge axis Y. The parallel walls 61, 62 are made for example of a dielectric material like for example a plastic material. When the cartridge 14 is received into the payload compartment 38 of the aerosol generation device 12, the cartridge axis Y coincides with the device axis X and the parallel walls 61, 62 of the cartridge housing 51 are in contact with the parallel walls 41, 42 of the payload compartment 38. Particularly, in this case, the wall 61 is in contact with the wall 41 and defines an airflow inlet facing the corresponding hole of the wall 41 to allow entering the airflow into in the cartridge 14. Similarly, the wall 62 is in contact with the wall 42 and defines an airflow outlet facing the corresponding hole of the wall 42 to allow evacuating the airflow from the cartridge 14.

In the example of figure 1, the cartridge housing 51 presents a rectangular shape. In this case, it further defines at least a pair of side walls 63, 64 extending between the parallel walls 61, 62 along the cartridge axis Y. According to another example, the cartridge housing 51 presents any other shape as for example a cylindrical shape extending along the cartridge axis Y. In this case, the cartridge housing 51 defines at least one side wall extending between the parallel walls 61, 62 along the cartridge axis Y. The or each side wall 63, 64 is made partially of a dielectric material, for example the same material as the parallel walls 61, 62. The walls 61 to 64 of the cartridge housing 51 delimit a storage portion 66 configured to store the aerosol forming precursor.

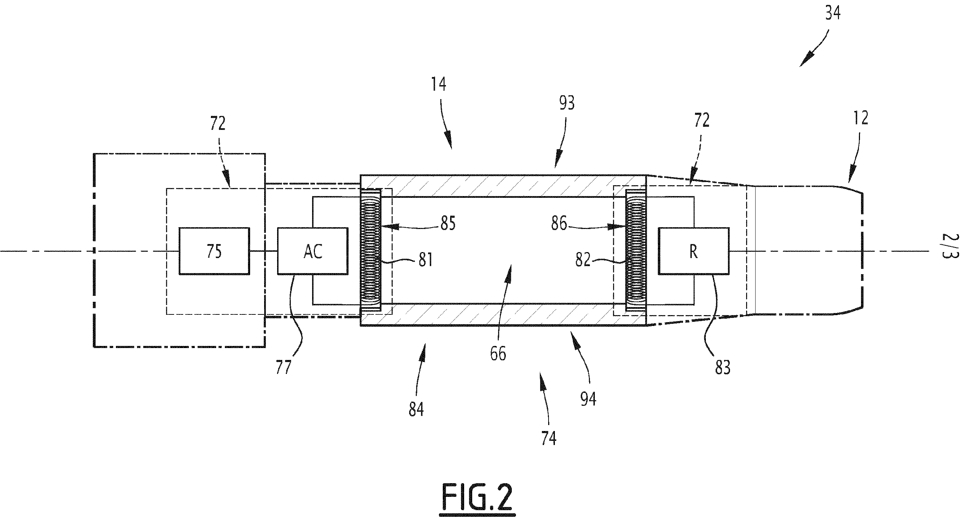

Figure 2 shows in more detail the heating system 34 in case when the cartridge 14 is received into the payload compartment 38 of the aerosol generation device 12. Referring to this Figure 2, the heating system 34 comprises a fixed part 72 integrated into the aerosol generation device 12 and a removable part 74 integrated into the cartridge 14.

The fixed part 72 of the heating system 34 comprises a heating control module 75, an AC source 77, a fixed magnetic core part, a primary winding 81, a secondary winding 82 and a load 83.

The heating control module 75 is configured to control the operation of the heating system 34 by controlling the transmitting of the direct current provided by the battery to the AC source 77. The control performed by the heating control module 75 is based on input parameters like precursor temperature, operation time, flow rate, etc. and makes it possible to generate output parameters like power transfer amplitude or/and frequency intended to control the AC source 77. According to a particular embodiment of the invention, the heating control module 75 is integrated into the controller 36 and presents for example a program code dedicated to control the heating capacities of the aerosol generation assembly. According to another embodiment of the invention, the heating control module 75 is formed by a separated controller similar to the controller 36 explained above.

The AC source 77 is connected electrically to the battery through the heating control module 75 on the one hand and to the primary winding 81 on the other. The AC source 77 presents a DC/AC inverter configured to transform the direct current provided by the battery into an alternative current to power the primary winding 81, according to the output parameters generated by the heating control module 75.

The fixed magnetic core part presents a fixed part of a magnetic core 84 and is composed of a first pair of parallel sides 85, 86 made of a magnetic material. The parallel sides 85, 86 are arranged perpendicularly to the device axis X. The parallel side 85 protrudes from the wall 41 of the storage compartment 38 adjacent to the battery end 22. The parallel side 86 protrudes from the wall 42 of the storage compartment 38 adjacent to the mouthpiece end 24.

The primary winding 81 is wound around the side 85 of the fixed magnetic core part and the secondary winding 82 is wound around the side 86 of the fixed magnetic core part. Thus, the primary winding 81 is adjacent to the battery end 22 and the secondary winding is adjacent to the mouthpiece end 24 of the device housing 21. The load 83 is connected electrically to the secondary winding 82 and presents for example a resistance with a resistance value R chosen to optimize a coupling effect between the two windings 81, 82. Particularly, the resistance value R is chosen to maximize the power transfer between the windings 81, 82. As it is visible on Figure 2, the load 83 can be arranged under the mouthpiece 40.

The removable part 74 of the heating system 34 comprises a removable magnetic core part of the magnetic core 84. Particularly, the removable magnetic core part is composed of a second pair of parallel sides 93, 94 made for example of the same material as the first pair of parallel sides 85, 86. The second pair of parallel sides 93, 94 is integrated into the side wall(s) 63, 64 of the cartridge housing 51 and designed to be in a tight contact with the first pair of parallel sides 85, 86 when the cartridge 14 is inserted into the payload compartment 38, to form a closed shape of the magnetic core 84. In other words, the second pair of parallel sides 93, 94 is designed to complete the first pair of parallel sides 85, 86 so as to form a closed shape magnetic core extending partially around the storage portion 66. In the example of Figure 2, the magnetic core 84 presents thus a rectangular shape.

Extremities of each of the parallel side 93, 94 of the second pair are adapted to cooperate with the corresponding extremities of the parallel sides 85, 86 of the first pair. The extremities of the parallel sides 93, 94 may extend beyond the corresponding side wall(s) 63, 64 and protrude from the corresponding parallel wall 61, 62 of the cartridge housing 51. Additionally, these extremities may for example form a chamfrain structure or a step structure to ensure a better contact with the corresponding extremities of the parallel sides 85, 86.

The parallel sides 93, 94 are for example integrated into the side wall(s) 63, 64 of the cartridge housing 51 to form at least one contact surface in contact with the aerosol forming precursor contained into the precursor storage 66. In other words, the contact surfaces of the parallel sides 93, 94 of the magnetic core 84 delimits partially the precursor storage 66.

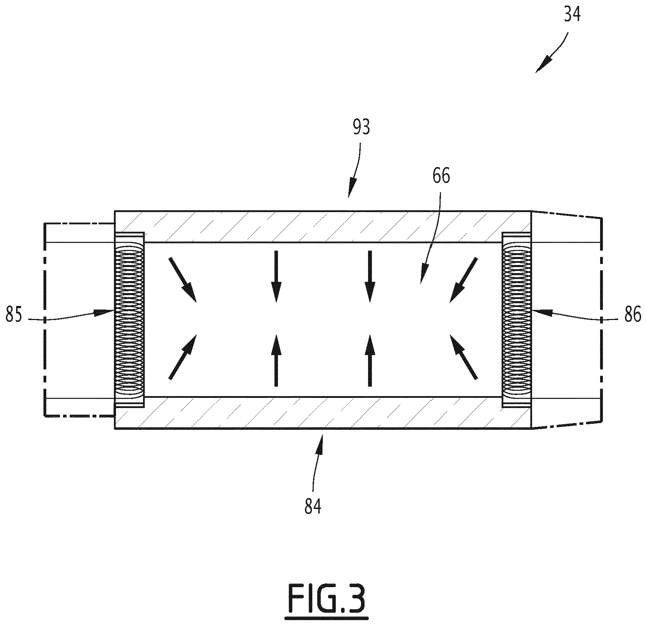

The operation of the aerosol forming assembly 10 will now be explained. Initially, it is considered that the cartridge 14 is extracted from the aerosol generation device 12. The cartridge can be for example purchased separately from the aerosol generation device 12 and used as a consumable. When the user is intending to activate the operation of the assembly 10, he/she inserts first the cartridge 14 into the payload compartment 38 of the aerosol generation device 12. Thus, the second pair of parallel sides 93, 94 comes into contact with the first pair of parallel sides 85, 85 and the magnetic core 84 is formed. Then, the user activates the operation of the controller 36 by activating for example a switch or by making a puff. This activates the operation of the heating control module 75 which controls the AC source 77 to power the primary winding 81. An AC current passing through the primary winding 81 induces and an electric current on the secondary winding 82. Foucault's currents (also called eddy currents) appear in the magnetic core 84. At least a part of these currents is transformed into heat which is transmitted to the precursor by direct contact with the contact surfaces of the magnetic core 84 as it is showed on Figure 3. Then, basing on the input parameters, the heating control module 75 may adjust the operation of the AC source 77 to achieve for example an optimized temperature of the precursor.

Other embodiments of the heating system 34 according to the invention are also possible. For example, different components of the system 34 may be arranged differently in respect with the fixed or removable parts. Particularly, the removable part of the heating system 34 may comprise other components, as for example the primary and/or the secondary windings. For example, when a mouthpiece is integrated with the cartridge, the secondary winding and the load may also be integrated into the cartridge. The heating system according to the invention may also be used in an aerosol generation device comprising a fixed precursor storage portion. In this case, the heating system 34 comprises only a fixed part with a fixed magnetic core arranged partially around the precursor storage portion. In both cases, the shape of the magnetic core may vary depending on the design of the aerosol generation device.

## Claims

1. A heating system (34) for an aerosol generation assembly (10) comprising an aerosol generation device (12) and a cartridge (14) designed to operate with the aerosol generation device (12), the aerosol generation device (12) comprising a battery and the cartridge (14) comprising a storage portion (66) for storing an aerosol forming precursor and a cartridge housing (51) delimiting the storage portion (66);
the heating system (34) comprising:
- a magnetic core (84) disposed at least partially around the storage portion (66);
- a primary winding (81) and a secondary winding (82) wound around the magnetic core (84) on opposite sides of the magnetic core (84);
- a load (83) connected electrically to the secondary winding (82);
- an AC source (77) connected electrically to the primary winding (81) and able to generate an alternative current;
- a heating control module (75) connecting the battery and the AC source (77), and configured to control the operation of the AC source (77) to generate aerosol by heat transfer from the magnetic core (84) to the aerosol forming precursor contained in the storage portion (66).

2. The heating system (34) according to claim 1, wherein the heating control module (75) is configured to control the operation of the AC source (77) based on the precursor temperature and/or the time of operating and/or the rate of the aerosol flow.

3. The heating system (34) according any one of the preceding claims, wherein the magnetic core (84) forms a closed shape.

4. The heating system (34) according to claim 3, wherein the magnetic core (84) forms a rectangular shape comprising a first pair of parallel sides (85, 86) and a second pair of parallel sides (93, 94).

5. The heating system (34) according to any one of the preceding claims, wherein the magnetic core presents a fixed magnetic core part integrated into the aerosol generation device (12) and a removable magnetic core part integrated into the cartridge (14), the removable magnetic core part being in connection with the fixed magnetic core part when the cartridge (14) is inserted into the aerosol generation device (12).

6. The heating system (34) according to claim 5, wherein the removable magnetic core part forms at least partially the cartridge housing (51) and presents a contact surface in contact with the aerosol forming precursor.

7. The heating system (34) according to claim 5 or 6, wherein the primary winding (81) and the secondary winding (82) are wound around the fixed magnetic core part.

8. The heating system (34) according to any one of claims 5 to 7 taken in combination with claim 4, wherein the fixed magnetic core part and the removable magnetic core part form respectively the first pair of parallel sides (85, 86) and the second pair of parallel sides (93, 94).

9. The heating system (34) according to any one of the preceding claims, wherein the primary winding (81), the secondary windings (82), the load (83), the AC source (77) and the heating control module (75) are integrated into the aerosol generation device (12).

10. A cartridge (14) designed to operate with an aerosol generation device (12) and comprising:
- a storage portion (66) for storing an aerosol forming precursor;
- a cartridge housing (51) delimiting the storage portion (66);
- a removable magnetic core part of the magnetic core (84) of the heating system (34) according to any one of the preceding claims.

11. The cartridge (14) according to claim 10, wherein:
- the cartridge housing (51) is extending along a cartridge axis (Y);
- said removable magnetic core part is integrated into the cartridge housing (51) and extends along the cartridge axis (Y).

12. An aerosol generation device (12) designed to operate with a cartridge (14) and comprising:
- a battery;
- a fixed magnetic core part of the magnetic core (84) of the heating system (34) according to any one of claims 1 to 9.

13. The aerosol generation device (12) according to claim 12, wherein:
- the aerosol generation device (12) is extending along a device axis (X) coinciding with a cartridge axis (Y) when a cartridge (14) is inserted into the aerosol generation device (12);
- said fixed magnetic core part extends perpendicularly to the device axis (X).

14. An aerosol generation assembly (10) comprising:
- a cartridge (14) according to claim 10 or 11;
- an aerosol generation device (12) according to claim 12 or 13.
